# EUROPEAN PATENT APPLICATION

(11) **EP 3 763 329 A1**
(43) Date of publication of application: **13.01.2021**
(21) Application number: 19185007.2
(22) Date of filing: 08.07.2019
(51) Int. Cl.: A61F 2/24, A61M 25/01

(54) **A MEDICAL ARRANGEMENT FOR INTRODUCING AN OBJECT INTO AN ANATOMICAL TARGET POSITION**

(71) Applicant: Medtentia International Ltd Oy, 02600 Espoo (FI)
(72) Inventor: Keränen, Olli, 23741 Bjärred (SE)
(74) Representative: Berggren Oy, Helsinki & Oulu

(57) **Abstract**

A medical arrangement (100) for introducing an object, such as an implant (110) into an anatomical target position (20) comprises a first introducer (101), a guide wire (103) and a third introducer (104). The guide wire (103) is configured to be introduced before the third introducer (104) and object (110) into or towards the anatomical target position (20). The third introducer (104) is configured to be delivered into the position along the guide wire (103), after which the guide wire (103) is configured to be retracted. The object (110) is configured to be delivered inside the third introducer (104) after the guide wire (103) is retracted.

## Description

### TECHNICAL FIELD OF THE INVENTION

The invention relates to a medical arrangement for introducing an object, such as an implant into an anatomical target position, such as a cardiac implant (like an annuloplasty medical device) into an annulus of a heart valve, such as a mitral valve or tricuspid valve.

### BACKGROUND OF THE INVENTION

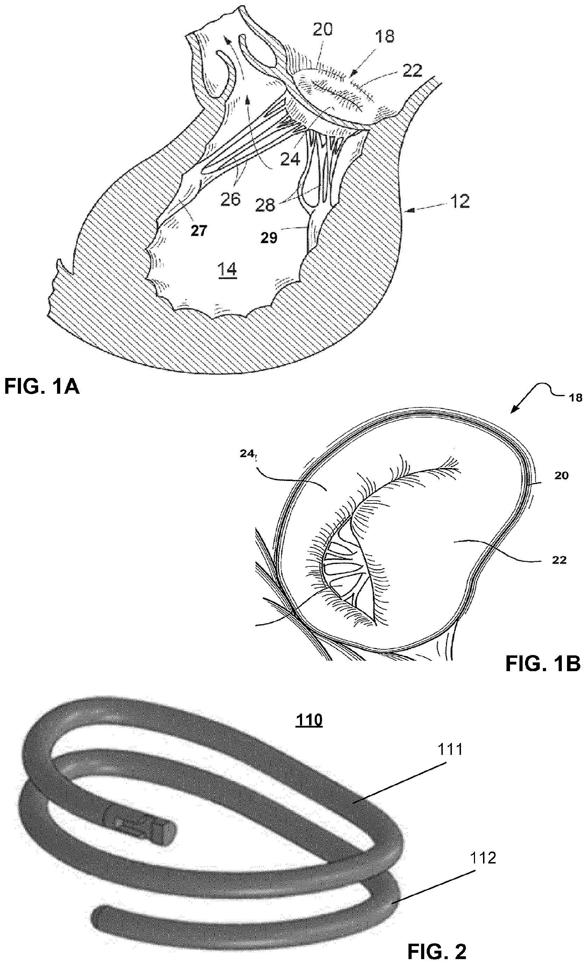

Fig. 1A illustrates a portion of the heart 12, the mitral valve 18, and the left ventricle 14 as an example of the anatomical target position. The mitral valve is at its boundary circumferenced by an annulus 20. The valve has two cusps or leaflets 22, 24. Each of these cusps or leaflets 22, 24 are connected to a respective papillary muscle 27, 29 via their respective connecting chordae 26, 28. In normal healthy individuals the free edges of the opposing leaflets will close the valve by coaptation. However, for some individuals the closure is not complete, which results in a regurgitation, also called valvular insufficiency, i.e. back flow of blood to the left atrium making the heart less effective and with potentially severe consequences for the patient. Fig. 1B illustrates a mitral valve 18, in which the leaflets 22, 24 do not close properly. This commonly occurs when the annulus 20 becomes dilated. One surgical procedure to correct this is to remove a portion of the leaflet 24 and stitch the cut edges together with one another. The procedure will pull back the annulus 20 to a more normal position. However the strength of the leaflet 24 is altered. Similar problems with a less effective heart function occur if one or both leaflets are perforated to such an extent that blood is flowing towards the left atrium, although the leaflets close properly.

In some conditions of degenerated heart function, the leaflets do not present a solid surface, as in a degenerative valve disease. The leaflet may also be ruptured, most commonly at an edge of a leaflet, resulting in an incomplete coaptation. Hence, cardiac devices and methods are developed for repairing of one or more leaflets of a heart valve, or other related anatomical structures, such as the chordae attached to the ventricular side of leaflets.

Figure 2 illustrates an exemplary implant to be delivered and introduced into an anatomical target position, and in particularly a cardiac implant 110. The implant may comprise one or more loop-shaped structures 111, 112. Advantageously one first loop-shaped structure is configured to abut a first side of the heart valve and one second loop-shaped structure is configured to abut a second, opposite, side of the valve to thereby trap a portion of the valve tissue 20 between the second and the first support structures 111, 112.

The implant is typically delivered via a catheter and has thus typically a delivery state, where the implant has an elongated form. In said delivery state the implant can be transferred advantageously through a catheter having diameter 7-10 mm, for example. The implant comprises typically a shape memory material having a first shape, such as the elongated form of the delivery state in a first temperature, and the second shape, such as the loop-shaped form in a second temperature. The second temperature corresponds advantageously essentially the body temperature, whereupon the implant takes the second shape, corresponding the loop-shaped form, when introduced for example with the blood flow in the atrium.

In addition, some problems arise due to a catheter system having both inner and outer catheters, usually steerable catheters, sometimes numerous inner catheters, where the inner catheter(s) locating inside the outer catheter limit(s) space from the implant.

It is found that the prior art cardiac implants, such as depicted above, work very well, but there are still some disadvantages relating to the introduction devices, such as catheter type devices, to deliver the implant into the anatomical target position, such as into the annulus of the heart valve. The catheter based systems are based for delivering a relative thick main catheter having a first curve portion into a first portion of the anatomical target position, such as to an atrium, then a second catheter having a second curve portion into a second portion of the anatomical target position, such as next to annulus or leaflets of the heart valve, and then a third catheter having a third curve portion into a third portion of the anatomical target position, such as around the annulus of the heart valve. In some systems there might be even further catheters to with further curve portions to be delivered before the implant can be delivered and introduced into its position. The third or further catheter if used, is called as a delivery catheter. The implant is then delivered to its position inside the feeding catheters, which is delivered to its position inside the other catheters.

There are some drawbacks related to the prior art catheter based systems, such as at least the main catheter must be thick (7-10 mm or even more) so that it can carry the further catheters inside. In addition, also the delivery catheter must be relative thick so that the implant can be delivered inside the delivery catheter. When the catheters are relatively thick, it is very hard to insert the catheters into the anatomical target position. For example, a sub-annular space below the annulus, so between the chordae and wall or septum, is very narrow, whereupon the best channel for the thick catheters is very difficult to find and deliver, in particularly when the maneuverability and steerability of the catheters is poor. Furthermore, the surface of the inner wall is rough, having additionally numerous attachment points of chordae, which easily causes stuck of the catheters. Additional challenges arise when the catheters, especially also the delivery catheter, have memory properties or predetermined shapes, which might activate too early and thereby raising possibility to stuck the catheter into the wall or other structures of the anatomical target position, such as the heart. Thus, the time limit to insert the catheters, in particularly the delivery catheter, having memory properties, is very limited so that the catheters can be inserted into their right and accurate position before the memory property will be activated by the temperature of the anatomical target position, such as the heart.

### SUMMARY OF THE INVENTION

It is an object of the invention to alleviate and eliminate the problems relating to the known prior art. Especially the object of the invention is to provide a medical arrangement for introducing an implant into an anatomical target position in an easy, fast, safe and accurate manner with a high degree of control. In addition, the object of the invention is to minimize the sizes of the catheters used and at the same time minimize stuck of the catheters and thereby minimize stress introduced for the anatomical target position.

The object of the invention can be achieved by the features of independent claim.

The invention relates to a medical arrangement for introducing an implant into an anatomical target position, such as a cardiac implant into an annulus of a heart valve, according to claim 1.

A medical arrangement according to the invention is configured to introduce an object, such as an implant, and in particularly such as a cardiac implant, or an annuloplasty medical device, from a distal end of the arrangement into an anatomical target position, such as into an annulus of a heart valve. The heart valve may be a mitral valve or tricuspid valve, for example, not limiting to those only. It is to be understood that the object can be also some other object, such as medicine, for example.

According to an example the object is the implant, which comprises in a use a loop shaped support portion, having either one or more loops or coils so that at least one first loop-shaped structure can be configured to abut a first side of the heart valve and at least one second loop-shaped structure to abut a second, opposite, side of the valve to thereby trap a portion of the valve tissue between the second and the first support structures. It is also possible that there is only the one first loop-shaped structure, which is configured to abut a first side of the heart valve, and not the second support structures, or vice versa. The implant is advantageously adapted to support for a mitral valve upon being fully delivered.

According to an embodiment the medical arrangement comprises a first introducer, a third (delivering) introducer, and a guide wire. The first introducer is advantageously an outer steerable catheter, which is delivered for example to the atrium or ventricle, but not to the anatomical target end position as such, where the implant is to be introduced. The third introducer is arranged to be operable between the first introducer and the guide wire. In addition, at least a distal portion of the third introducer is configured to be introduced from the distal end portion of the first introducer after the guide wire is at least partially introduced into or towards said anatomical target position.

According to an embodiment the guide wire is configured to be introduced into or towards said anatomical target position via the first introducer but before the implant, as well as before the third introducer (meaning that the guide wire is delivered beforehand so that it can guide especially the delivery of the third introducer). Also, the third introducer is configured to be introduced into or towards said anatomical target position before the implant. When the guide wire is delivered into the right position, the third introducer is then delivered along the guide wire and guided by the guide wire (and inside the first introducer) into the anatomical target position.

The implant is configured to be delivered and instructed inside the third introducer (and inside the first introducer) into or towards said anatomical target position after at least the distal portion of the third introducer is introduced from the distal end portion of the first introducer into or towards said anatomical target position. Advantageously the implant is configured to be delivered and instructed inside the third introducer after the distal portion of the third introducer has reached the anatomical target position, even if the delivering of both the third introducer and the implant can be made sequentially and periodically. However, and advantageously, the implant is delivered only after the guide wire is retracted away from said anatomical target position, but so that the third introducer into is still left into said anatomical target position). By this more space can be left for the implant when delivered inside the third introducer. In addition, when the guide wire is used for guiding the third introducer, more flexible and thinner third introducer can be used, because the third introducer does not need to be any controllable or steerable catheter, for example. It is enough that said third introducer can follow the guide wire towards or into the anatomical target position, and protect the tissue and prevent possible jamming of the implant to the tissue during introduction of the implant, and if the object to be delivered it e.g. liquid medicament, the third introducer enables the delivering of the liquid medicament to the target position.

The guide wire has advantageously an activated shape and an inactivated shape, wherein in said inactivated shape the guide wire can be delivered in a straightened configuration through the first introducer and in said activated shape the guide wire takes at least a first curved shape within or near the anatomical target position, such as in the atrium or ventricle or proximate the mitral valve. The guide wire advantageously has a preformed shape so to take the activated shape when introduced to the anatomical target position and introduced from the distal end of the first introducer. According to an embodiment the guide wire is at least partially formed from a shape memory material and thereby operable to assume said activated shape when meet the temperature of the anatomical target position. The activated shape can be achieved also via another techniques known by the skilled person. When the guide wire is activated to said at least first curved shape, it is delivered to the anatomical target position, after which the third introducer is delivered along and guided by the guide wire to the anatomical target position, as is described in this document.

In addition, the distal end of the guide wire may comprise a curvature tip portion, such as a J-shape, in order to allowing smooth delivery of the distal end of the guide wire and to prevent the distal end of the guide wire to get tangled in to tissue. The curvature tip portion may have a preformed shape or being at least partially formed from a shape memory material and thereby taking the shape of the curvature tip portion when introduced into the anatomical target position.

According to an embodiment the third introducer is configured to take the shape of the at least first curved shape of the guide wire when it is delivered along the guide wire (which already has taken the first curved shape) and remain said first curved shape after the guide wire is retracted away from said anatomical target position before delivering the implant. The implant is then configured to follow said third introducer and said first curved shape into said anatomical target position during introduction and delivering.

The implant may comprise a hollow or solid tubular structure. The implant can be delivered into said anatomical target position so that it travels inside the third introducer (advantageously after the guide wire is retracted). This offers clear advantages over the known prior art solutions namely because the guide wire is very thin compared to the catheters, it is remarkable easy, fast, safe and accurate to insert the third introducer, even if it is flexible and soft, via very small and narrow channels of the anatomical target position to the correct place. When the third introducer is delivered to the correct position and the guide wire is removed, the implant can be easily delivered along and inside the third introducer to the correct end position.

The implant can be made from metal, but also fabric, polyurethane or polyester and there is no need for the implant to have any memory property.

The implant can be delivered to the position for example by a pusher coupled to the proximal end of the implant, whereupon the implant can be pushed to the position by the pusher. In the case of non-rigid implant, for example if the implant is made of textile or the like, the pusher can be coupled to the distal end of the implant and thereby to draw the implant to the position.

According to an embodiment the implant may have at least a first curved shape having a preformed shape and capable of being delivered in a straightened configuration through the first introducer, whereupon the implant is activated to at least a first curved shape within or near the anatomical target position, such as in the atrium or ventricle proximate the mitral valve of the heart. The implant may comprise e.g. a shape memory property and biased to said curved shape. However, it is to be noted that according to the invention, in particularly the implants without any memory property can be delivered and guided by the guide wire, which is delivered to the position first.

It is to be noted that the implant is configured to follow said third introducer and said first curved shape of the third introducer into said anatomical target position. When the implant has been introduced into said anatomical target position, also the third introducer can be retracted and the implant essentially maintains the shape taken when introduced into said anatomical target position, so the shape of the guide wire and the third introducer in the anatomical target position. It is to be noted that the implant can be attached, such as sutured, into its position for example by keeping the implant in its position during the attaching by the third introducer in the case, where the third introducer is only partially retracted to uncover. The implant can also be as a self-attaching version having e.g. teeth, whereupon when the introducer over the implant is retracted the teeth can dig into the tissue and thereby attach the implant into its position.

In addition, when the implant is inside the third introducer and delivered to the anatomical target position, the position of the implant can still be adjusted by manipulating the third introducer, such as drag the third introducer and thereby adjust the position or orientation of the implant (a whole packet of the third introducer and the implant), which is very advantageous in many situations. In addition, if something goes wrong, the implant can still be retracted away by the third introducer.

According to an embodiment the arrangement may additionally comprise a second introducer, such as a catheter and in particularly an inner steerable catheter. The second introducer is arranged to be operable between the first introducer and the guide wire. The second introducer is configured to be introduced from the distal end portion of the first introducer. According to an embodiment, the second introducer is an optional introducer and can be used for example to bypass the leaflets of the heart or other anatomical portion in or near the target position, as well as to achieve am additinal turn to a specific direction by turning the distal end of the second introducer (namely turnability of the introducers are limited). However, according to an embodiment at least a distal portion of the second introducer is configured to be introduced from the distal end portion of the first introducer before the guide wire, and thereby guide or instruct the guide wire to bypass for example a certain anatomical portion, such as leaflets.

If the second introducer is used, the implant can be delivered inside the second introducer (and inside the first introducer), and again inside the third introducer into or towards the anatomical target position after at least the distal portion of the second introducer is introduced from the distal end portion of the first introducer.

However, the second introducer is advantageously configured to be retracted after said guide wire and the third introducer are introduced into said anatomical target position and before said implant is introduced into said anatomical target position. This is not mandatory but by this a more space can be provided for the implant to the third introducer, and in particularly when the third introducer is flexible and expandable so that the diameter of third introducer can expand when the implant is delivered.

When the implant is delivered into the anatomical target position, the third introducer is configured to be retracted away so that said implant essentially maintains the shape taken when introduced into said anatomical target position. The third introducer is configured to be retracted either at once or sequentially, thereby uncovering the implant, which can be then secured, such as sutured or stapled for example, to the anatomical target position.

Still in addition, the arrangement may also comprise a cooling arrangement for cooling the guide wire for example during retracting the guide wire, but also in other phases. When the guide wire is cooled down it can be easily reshaped and for example retracted out essentially easily transformable state. The cooling arrangement may be arranged so that there is an inlet for the cooling agent, such as cool water, in the proximal end of the first, second and/or third introducer, whereupon the cooling agent can flow between the walls of the first, second and/or third introducer from the proximal ends towards the distal ends, for example. According to an embodiment the distal ends may have openings so that the cooling agent can flow out.

The present invention offers advantages over the known prior art, such as an easy, safe, precise and time saving manner to reliable delivering the implant to the anatomical target position such as to the annulus of the valve. Still, the guide wire is very convenient to deliver to the target position, namely it is very thin compared to the catheters and thus it does not tangle to the tissue. When the guide wire is delivered into the target position, the subsequent catheters, even flexible catheters, can be delivered easily along and guided by the guide wire. In addition, the third introducer can be used as a rescue device during securing the object, like the implant, namely if something goes wrong in securing, any device used for pulling out the implant can be delivered to the proximal end of the implant along and guided by the third introducer, because the third introducer can be kept in the target position or at least to extend over the proximal end portion of the implant as long as the implant is secured.

In addition, the present invention provides for a compact arrangement for delivering the implant. The compact medical device allows minimally invasive procedure. Furthermore, when using the compact catheter-operated medical device, risks for having any medical drawbacks or symptoms are much lower than e.g. in the traditional open-heart operation. Also, the patient recovery process is much faster.

The exemplary embodiments presented in this text are not to be interpreted to pose limitations to the applicability of the appended claims. The verb "to comprise" is used in this text as an open limitation that does not exclude the existence of also un-recited features. The features recited in depending claims are mutually freely combinable unless otherwise explicitly stated.

The novel features which are considered as characteristic of the invention are set forth in particular in the appended claims. The invention itself, however, both as to its construction and its method of operation, together with additional objects and advantages thereof, will be best understood from the following description of specific example embodiments when read in connection with the accompanying drawings.

### BRIEF DESCRIPTION OF THE DRAWINGS

Next the invention will be described in greater detail with reference to exemplary embodiments in accordance with the accompanying drawings, in which:
- Figures 1A-1B: illustrate schematically a portion of a heart and mitral valve,
- Figure 2: illustrates an exemplary tissue anchoring unit for securing the flexible leaflet to the adjacent tissue and/or annulus according to an advantageous embodiment of the invention, and
- Figures 3-27: illustrate a medical arrangement for introducing an implant into an anatomical target position according to advantageous embodiments of the invention.

### DETAILED DESCRIPTION

Figures 1A-1B and 2 are already discussed in more details in connection with the background of the invention portion above.

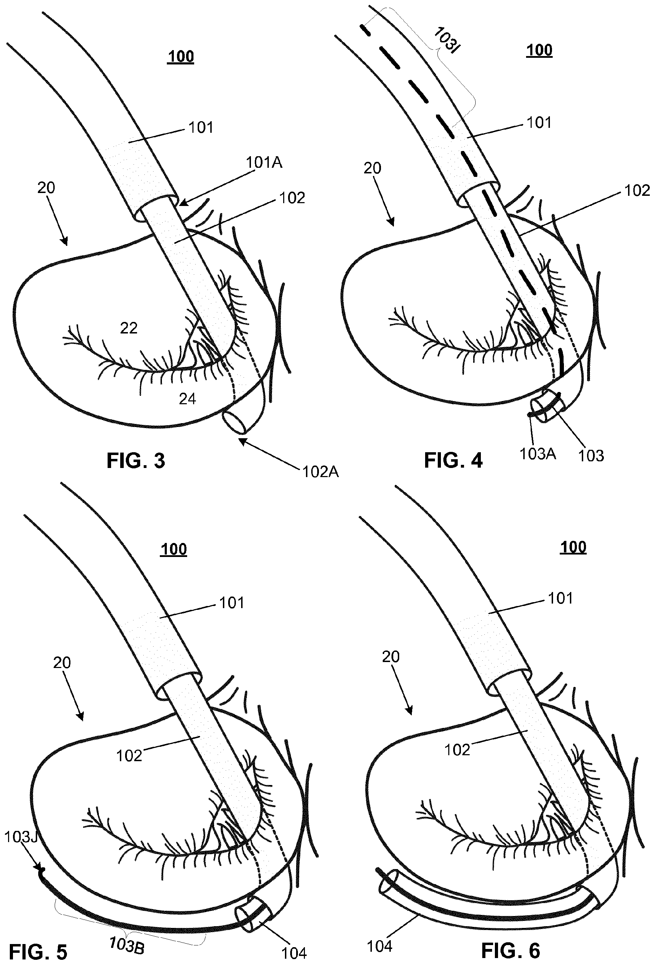

Figures 3-27 illustrate a medical arrangement for introducing an implant into a heart as an example of the anatomical target position according to advantageous embodiments of the invention. It is to be noted that also other kinds of object in addition to the implant can be delivered in the similar manner, such as medicines, for example. In Fig 3 the first introducer 101 is delivered next to the annulus 20 of the heart, and the second introducer 102 is introduced from the distal end portion 101A of the first introducer 101. It is to be noted that the second introducer is an optional introducer, but it is still illustrated in Figures as an example. The second introducer can be used for example to bypass the leaflets 22, 24 of the heart, or other anatomical portion, as well as to achieve a turn to a specific direction by turning the distal end 102A of the second introducer 102 (namely turnability of the introducers are limited). In addition, it is to be noted that at least a distal portion 102A of the second introducer 102 is introduced from the distal end portion 101A of the first introducer 101 before the guide wire, and thereby guide or instruct the guide wire 103 to bypass the leaflets, for example, as can be seen in Fig 4.

When the second introducer has bypassed the leaflets 22, 24, the guide wire 103 can be delivered into or towards the position 20. It is to be noted that the guide wire 103 is delivered into the position before the implant 110 and third introducer 104.

The guide wire 103 has advantageously an activated shape and an inactivated shape, wherein in said inactivated shape the guide wire 103 can be delivered in a straightened configuration 1031 through the first introducer 101, as can be seen in Fig. 4. In said activated shape the guide wire 103 takes at least a first curved shape 103B within or near the anatomical target position 20. When the guide wire 103 is activated to said at least first curved shape 103B, it is delivered to the position. According to an example the distal end 103A of the guide wire 103 may comprise a curvature tip portion 103J, such as a J-shape, in order to allowing smooth delivery of the distal end 103A of the guide wire 103 and to prevent the distal end 103A of the guide wire 103 to get tangled in to tissue.

The arrangement advantageously comprises also a third introducer 104, as can be seen in Figs. 5-11, where the third introducer 104 is operated between the first introducer 101 and the guide wire 103, and if the second introducer is used, also between the second introducer 102 and the guide wire 103. The third introducer 104 is introduced from the distal end portion 101A of the first introducer 101 (and from the distal end portion of the second introducer, if used). It is to be noted that at least a distal portion 104A of the third introducer 104 is introduced from the distal end portion 101A of the first introducer 101 (and from the distal end portion 102A of the second introducer 102, if used) only after the guide wire 103 is introduced into or towards said anatomical target position 20.

The third introducer 104 is delivered along the guide wire 103 into or towards said anatomical target position 20 after the guide wire 103 is at least partially introduced from the distal end portion 101A of the first introducer 101, as can be seen in Figs. 5-8. Most advantageously the third introducer 104 is delivered to the anatomical target position 20 when the guide wire is fully delivered to the anatomical target position, in particularly when the third introducer 104 is flexible.

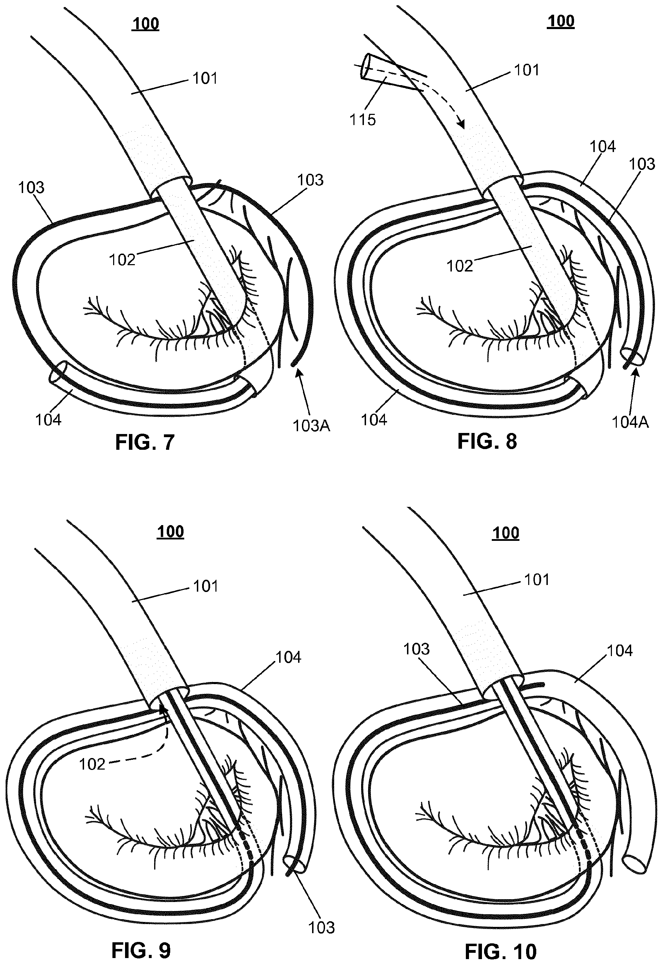

When the guide wire 103 and the third introducer 104 are delivered into the position, the second introducer 102 is retracted and it is retracted advantageously before delivering the implant 110 into the anatomical target position 20, as is the case in Fig. 9. This is not mandatory but by this a more space can be arranged for the implant.

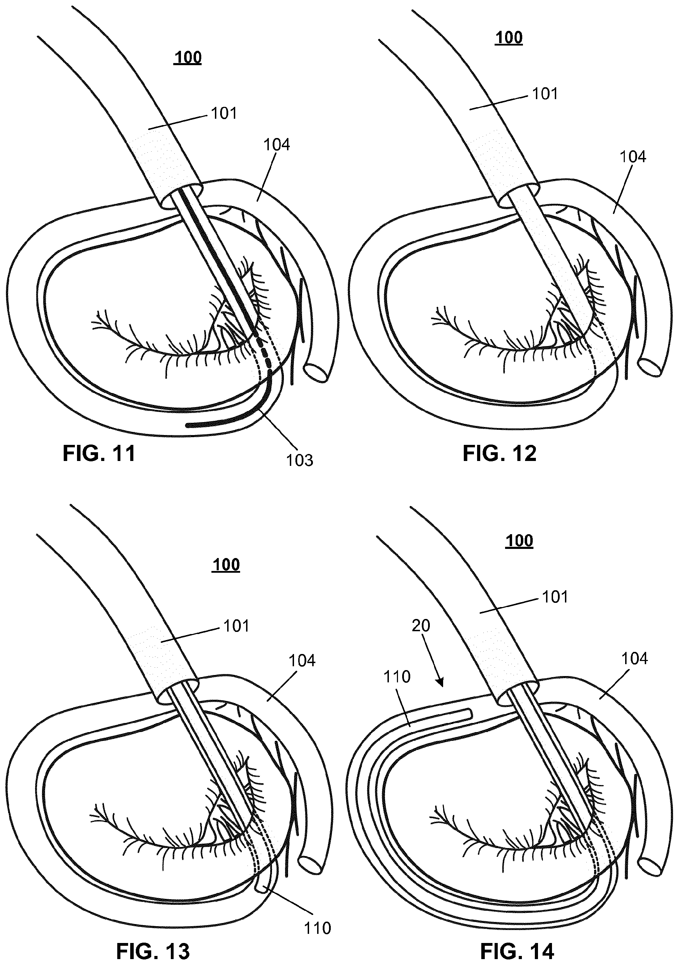

In addition, after the third introducer 104 has been delivered into the anatomical target position, the guide wire 103 can be retracted, as can be seen in Figs. 10-12. After removing the guide wire 103, the implant 110 is delivered inside the third introducer 104 into or towards said anatomical target position, as can be seen in Figures 13-15.

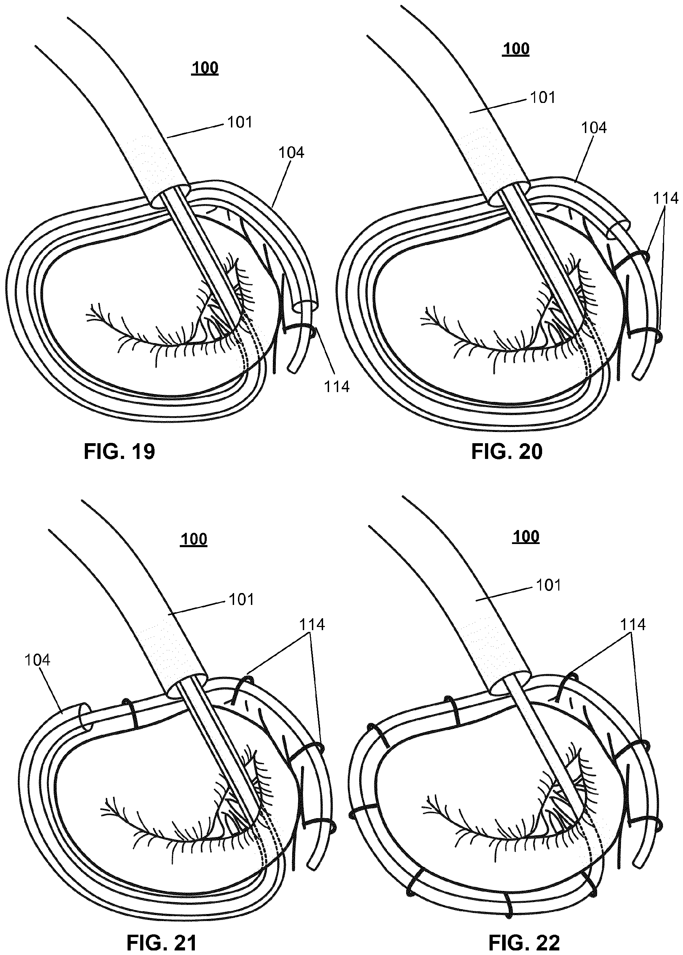

The third introducer 104 can be retracted after the implant 110 has been introduced into the anatomical target position 20. After removing the third introducer 104, the implant can be secured to the tissue by securing members 114, as can be seen in Figs. 19-22. The securing can be done for example by suturing or stabling or by other securing methods known by the skilled person.

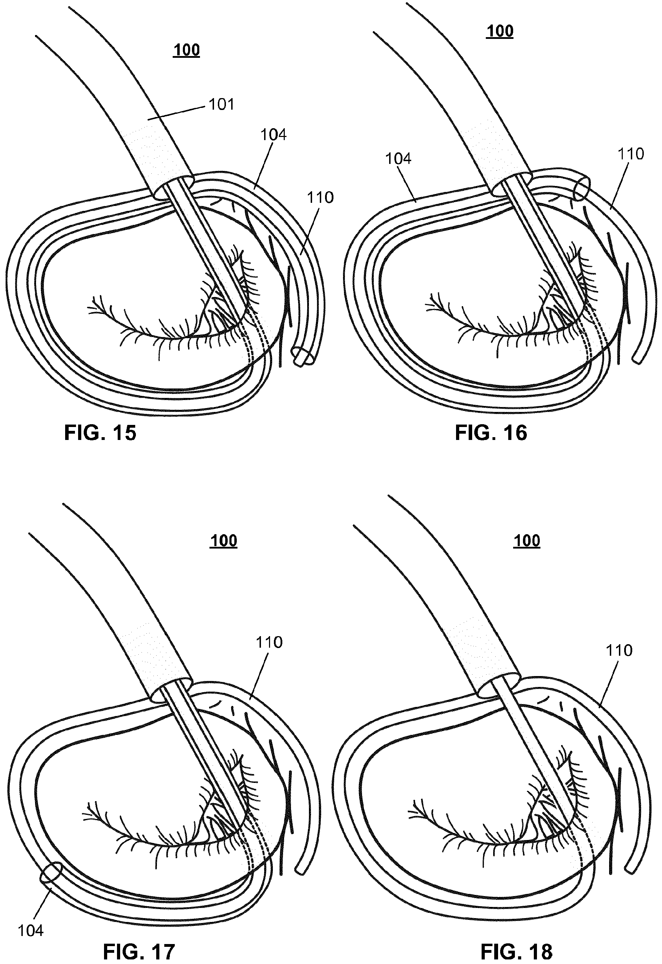

It is to be noted that the third introducer 104 can be retracted at once, as is the case in Figs. 16-18, or sequentially, as is the case in Figs. 19-22. When the third introducer 104 is retracted sequentially, only a part of the implant is uncovered by one move for securing. After securing said uncovered part the third introducer 104 can be retracted more, thereby uncovering additional portion of the implant for securing.

In addition, Fig. 8 illustrates also a cooling arrangement 115 for supplying the cooling agent (arrow) and thereby cooling the guide wire 103. It is to be noted that the arrangement may comprise one or more cooling arrangements 115 and arranged in connection with the first, second and/or third introducers. In addition, even if the cooling arrangement 115 is illustrated only in connection with Fig. 8, it should be understood, that it may also be comprised in other embodiments and arrangements 100 illustrated in other Figures.

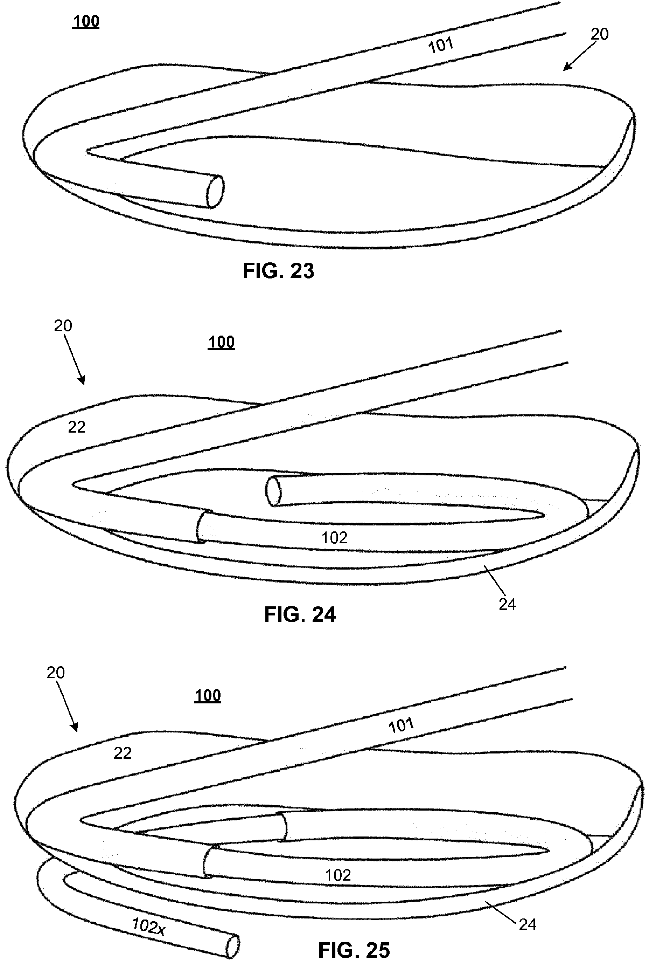

Figures 23-27 illustrate a further example of the medical arrangement 100 to introduce the object into the anatomical target position, and especially the implant 110 into a mitral valve 20 (as an example of the anatomical target position). When introducing the implant, at least one loop-shaped structure 111 of the implant 110 abuts a first side of the heart valve and at least one second loop-shaped structure 112 abuts a second, opposite, side of the valve to thereby trap a portion of the valve tissue 20 between the second and the first support structures 111, 112. In Figs. 23-27 the first introducer 110 has a first curve shape and the second introducer 102 has a second curve shape to the same curvature direction as the first curve shape of the first introducer 101 so to form a concentric system 100. As can be seen in Fig. 25, the arrangement may also have an additional second introducer 102x, which again has third curve shape but still to the same curvature direction as the first and second curve shapes of the first and second introducers, wherein said first, second and third curved shapes are concentric curved shapes. In addition, the additional second introducer 102x may also be a steerable catheter or having pre-curved structure so that it has ability to seek said third curve shape at least and advantageously when delivered into or towards the anatomical target position.

It is to be noted that the first introducer is introduced next to the annulus and the second as well as additional second introducers 102, 102x are used to bypass the leaflets 22, 24 and to be introduced to the opposite side of the annulus as the first introducer is delivered.

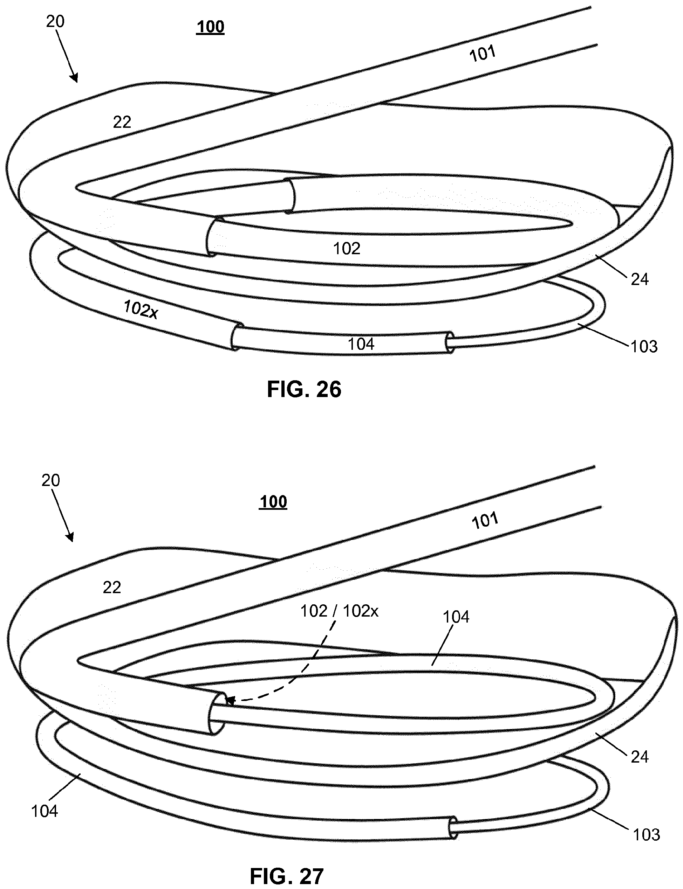

Figures 26 and 27 illustrate the arrangement 100, where the third introducer 104 and also the guide wire 103 are introducer into or at least towards the position for delivering the implant. As can be seen in Fig. 27, the second as well as additional second introducers 102, 102x can be retracted before introducing the implant. The implant and the steps for delivering it, as well as retracting the guide wire 103 before delivering the implant, are not shown, but the fundamental principles are the same as described elsewhere in this document.

It is to be noted that according to an embodiment the first introducer 101 can be retracted already after the second introducer 102 is delivered and before the delivery of the other introducers 102x, 104 and implant, and the second introducer 102 can be retracted after the additional second introducer 102x is delivered and before the delivery of the third introducer 104 and implant, and that the additional second introducer 102x can be retracted after the third introducer 104 is delivered toward or into the anatomical target position and before the delivery of the implant 110. In this way a maximum space can be provided for the delivering catheter 104, or the third introducer 104, and in particularly when the third introducer 104 in an expandable introducer 104, whereupon relatively big implant can be delivered into the anatomical target position. Previously, the diameter of the all additional introducers must have been smaller and smaller, whereupon the diameter of the last delivering catheter is particularly small, this remarkably limiting also the size of the object, such as the implant, to be delivered.

The invention has been explained above with reference to the aforementioned embodiments, and several advantages of the invention have been demonstrated. It is clear that the invention is not only restricted to these embodiments, but comprises all possible embodiments within the spirit and scope of the inventive thought and the following patent claims. For example the guide wire is at least partially formed from a shape memory material operable to assume an activated shape and an inactivated shape, wherein in said inactivated shape the guide wire is configured to be delivered in a straightened configuration through said first introducer and in said activated shape the guide wire is configured to take said at least first curved shape within or near the anatomical target position. The guide wire is advantageously configured to be introduced before the implant into or towards the anatomical target position. However, it is to be noted that the guide wire should at least reach the target position before the implant.

In addition, it is to be noted that even if the implant is described in this document as an example to be delivered, also other kinds of object can be delivered according to the invention, such as medicaments, for example. Furthermore, even if the heart is described in many embodiments, it is to be understood that the heart is only an example of the anatomical target. Still, in addition it is to be noted that the implant can be rigid or flexible.

## Claims

1. A medical arrangement (100) configured to introduce an object (110), such as an implant (110), from a distal end of the arrangement into an anatomical target position (20), the medical arrangement comprising a first introducer (101) and a guide wire (103), wherein
- said guide wire (103) has at least a first curved shape,
∘ said at least first curved shape having a preformed shape capable of being delivered in a straightened configuration (1031) through said first introducer (101), and
∘ configured to be activated to said at least first curved shape (103B) within or near the anatomical target position (20),
- at least a distal portion (103A) of the guide wire (103) is configured to be introduced from the distal end portion (101A) of the first introducer (101) before the object (110) into or towards said anatomical target position (20),
wherein
- the arrangement comprises a third introducer (104), where
∘ said third introducer (104) is arranged to be operable between the first introducer (101) and the guide wire (103), and
∘ at least a distal portion (104A) of the third introducer (104) is configured to be introduced from the distal end portion (101A) of the first introducer (101) into or towards said anatomical target position (20), and along and guided by the guide wire (103),
- said object (110) is configured to be delivered inside the third introducer (104) into or towards said anatomical target position (20) after at least the distal portion (104A) of the third introducer (104) is introduced from the distal end portion (101A) of the first introducer (101) into or towards said anatomical target position (20) and after the guide wire (103) is retracted away from said anatomical target position (20).

2. An arrangement of claim 1, wherein the third introducer (104) is configured to be delivered along the guide wire (103) into said anatomical target position (20) after the guide wire (103) is at least partially introduced from the distal end portion (101A) of the first introducer (101), whereupon said third introducer (104) is configured to follow said guide wire (103) and said first curved shape (103B) of the guide wire (103) into said anatomical target position (20).

3. An arrangement of claim 2, wherein said third introducer (104) is configured to take the shape of the at least first curved shape (103B) of the guide wire (103) and remain essentially said first curved shape (103B) after the guide wire (103) is retracted away from said anatomical target position (20).

4. An arrangement of claims 2-3, wherein said object (110) is configured to follow essentially said third introducer (104) and said first curved shape (103B) into said anatomical target position (20) during introduction.

5. An arrangement of any previous claims, wherein the arrangement comprises a cooling device (115) for cooling the guide wire (103) during retracting the guide wire (103).

6. An arrangement of any previous claims, wherein at least the distal portion (104A) of the third introducer (104) is configured to be introduced from the distal end portion (101A) of the first introducer (101) after the guide wire (103) is at least partially introduced into or towards said anatomical target position (20).

7. An arrangement of any previous claims, wherein said third introducer (104) is configured to be retracted after said object (110) has been introduced into said anatomical target position (20) so that said object (110), like the implant, essentially maintains the shape taken when introduced into said anatomical target position (20).

8. An arrangement of any previous claims, wherein the object (110), like the implant, is configured to be secured to the anatomical target position after the third introducer (104) is retracted at least partly.

9. An arrangement of any previous claims, wherein the arrangement additionally comprises a second introducer (102), where said second introducer (102) is arranged to be operable between the first introducer (101) and the third introducer (104) and/or guide wire (103).

10. An arrangement of claim 9, wherein the second introducer (102) is configured to be introduced from the distal end portion (101A) of the first introducer (101) and wherein at least a distal portion (102A) of the second introducer (102) is configured to be introduced from the distal end portion (101A) of the first introducer (101) before the guide wire and/or third introducer (104).

11. An arrangement of any previous claims, said first introducer (101) is a catheter, in particularly an outer steerable catheter (101), and/or wherein the third introducer (104) is a flexible catheter, and wherein the second introducer (102) of claim 9 or 10 is a catheter, in particularly a steerable catheter (102).

12. An arrangement of any of claims 9-11, wherein said object (110) is configured to be delivered inside the second introducer (102) into or towards said anatomical target position (20) after at least the distal portion (102A) of the second introducer (102) is introduced from the distal end portion (101A) of the first introducer (101).

13. An arrangement of any of claims 9-11, wherein said second introducer (102) is configured to be retracted after said guide wire (103) and/or third introducer (104) is introduced into said anatomical target position (20) and/or before said implant (110) is introduced into said anatomical target position (20).

14. An arrangement of any of previous claims, wherein the distal end (103A) of the guide wire (103) comprises a curvature tip portion (103J), such as a J-shape, in order to prevent the distal end (103A) of the guide wire (103) to get tangled in to tissue.

15. An arrangement of any of previous claims, wherein the object is the implant (110), said implant comprising a loop shaped support portion, having one or more loops or coils (111, 112) and adapted to support said anatomical target position, such as a mitral valve, upon being fully delivered, and wherein the implant (110) comprises a hollow or solid tubular structure, whereupon the implant (110) is configured to be delivered into said anatomical target position (20) so that it travels inside the third introducer (104).
